# EUROPEAN PATENT APPLICATION

(11) **EP 4 785 907 A1**
(43) Date of publication of application: **05.08.2026**
(21) Application number: 24890351.0
(22) Date of filing: 27.09.2024
(51) Int. Cl.: A61F 2/24, A61B 17/12

(54) **COATED MEDICAL DEVICE**

(30) Priority: 16.11.2023 CN 202311535198
(71) Applicant: Shanghai Microport Cardioadvent Co., Ltd., Shanghai 201203 (CN)
(72) Inventor: ZHOU, Ting, Shanghai 201203 (CN); GUO, Qingfang, Shanghai 201203 (CN); YAO, Yao, Shanghai 201203 (CN); LI, Junfei, Shanghai 201203 (CN)
(74) Representative: Marks & Clerk LLP
(86) International application number: PCT/CN2024/121619
(87) International publication number: WO 2025/102993

(57) **Abstract**

A covered medical device includes a main mesh stent (10) and a cover (20). The cover (20) covers at least part of an external surface of the main mesh stent (10) and comprises first and second friction zones, which are arranged along an axis of the main mesh stent (10) from a proximal end (101) to a distal end (102) thereof. The first friction zone has a COF lower than a COF of the second friction zone and extends from the proximal end (101) of the main mesh stent (10) and covers a maximum outer diameter position of the mesh stent (10). Through the first and second friction zones of the cover (20), this covered medical device provides good sheath loading performance and anchoring performance. It can be loaded into a sheath with less effort and provide high anchoring strength, without compromising other performance aspects.

## Description

### TECHNICAL FIELD

The present invention relates to the technical field of medical devices and, in particular, to a covered medical device providing both good sheath loading performance and anchoring performance.

### BACKGROUND

Occluders and other covered medical devices can be used as implants for left atrial appendage (LAA) closure and intervention of various diseases, such as atrial septal defect (ASD), patent ductus arteriosus (PDA), ventricular septal defect (VSD) and patent foramen ovale (PFO). Currently, there are various known occluder products, which can be categorized into three types: wire-braided ones, cut tubular ones and hybrids thereof. In order to attain effective occlusion, a polymeric covering membrane is typically disposed on an external surface or in interior of a main mesh stent (mesh-like scaffold). However, for an occluder with the covering membrane being disposed on an external surface, the friction between the covering membrane and the sheath is relatively high. During loading into and withdrawal from the sheath, the covering membrane at the location of the occluder's maximum outer diameter often contributes the greatest loading force. Such a large loading force places limitations both on expansion of the occluder' size and on minimization of the sheath's outer diameter. Most conventional solutions to this typically rely on modifying the structure of the occluder, reducing the thickness of the covering membrane, or increasing the sheath's inner diameter. However, all these solutions require compromising other performance aspects and therefore impose significant constraints on the structural design of the occluder. Further, a smaller loading force may mean less post-implantation stability of the occluder and hence an increased risk of potential safety hazards.

It should be noted that the information disclosed in this Background section is merely intended to provide a better understanding of the general context of the present invention and should not be taken as an acknowledgement or any form of admission that the information forms part of the common general knowledge of those skilled in the art.

### SUMMARY OF THE INVENTION

In view of this, the present invention seeks to provide a covered medical device, which can be loaded into a sheath with a smaller loading force and provide higher post-implantation stability, while not compromising other performance aspects.

To this end, the present invention provides a covered medical device comprising a main mesh stent and a cover. The cover covers at least a portion of an external surface of the main mesh stent and comprises a first friction zone and a second friction zone that are arranged along an axis of the main mesh stent from a proximal end to a distal end. The first friction zone has a coefficient of friction (COF) lower than a COF of the second friction zone and extends from the proximal end of the mesh stent and covers a maximum outer diameter position of the main mesh stent.

Optionally, the cover may comprise a covering membrane and a coating layer, the covering membrane covering the external surface of the main mesh stent, the coating layer covering the covering membrane, the coating layer comprising a first COF coating layer that forms the first friction zone.

Optionally, an area of the covering membrane that is not covered by the first COF coating layer is an uncoated area, wherein the uncoated area forms the second friction zone.

Optionally, the coating layer may further comprise a second COF coating layer that forms the second friction zone, wherein an area of the covering membrane that is not covered by the first COF coating layer is covered by the second COF coating layer.

Optionally, the first COF coating layer may cover the covering membrane, or the first COF coating layer may cover at least one first transition coating layer with a higher or a lower COF than the first COF coating layer.

Optionally, the second COF coating layer may cover the covering membrane, or the second COF coating layer may cover at least one second transition coating layer with a higher or a lower COF than the second COF coating layer.

Optionally, the first COF coating layer may comprise one or a combination of a fluoropolymer, Parylene, polyimide, polyvinylpyrrolidone, polyacrylamide, poly(ether amide) and a gel.

Optionally, the fluoropolymer may comprise one or a combination of polytetrafluoroethylene, polyvinylidene fluoride, a vinyl fluoride homopolymer and a perfluorinated ethylene propylene copolymer.

Optionally, the coating layer may have a thickness not exceeding 1 µm.

Optionally, the COF of the first friction zone may not exceed 0.3.

Optionally, the COF of the first friction zone may not exceed 0.1.

Optionally, the cover may consist of a covering membrane composed of a first membrane and a second membrane that have different COFs and are pieced together, wherein the first membrane forms the first friction zone, and the second membrane forms the second friction zone.

In summary, the present invention provides a covered medical device comprising a main mesh stent and a cover. The cover covers at least portion of an external surface of the main mesh stent and comprises a first friction zone and a second friction zone, which are arranged along an axis of the main mesh stent from a proximal end to a distal end. The first friction zone has a COF lower than a COF of the second friction zone and extends from the proximal end of the main mesh stent and covers a maximum outer diameter position of the main mesh stent.

With this design, during loading or unloading of the covered medical device into or from a sheath, the first friction zone can reduce the friction between the cover and the sheath, thereby enabling the cover at the maximum outer diameter position on the main mesh stent to have a lower loading force. Moreover, after the covered medical device is implanted in a body, the second friction zone can increase the friction between the cover and a target object, thereby enhancing the stability of the implanted device. Ultimately, the covered medical device of the present invention achieves both good sheath loading performance and anchoring performance, realizing low loading force and high anchoring force without compromising other aspects of the device's performance.

### BRIEF DESCRIPTION OF THE DRAWINGS

Those of ordinary skill in the art will understand that the following drawings are presented to enable a better understanding of the present invention and not intended to limit the scope thereof in any sense, in which:
Fig. 1 shows a schematic representation of a covered medical device according to embodiments of the present invention;
Fig. 2 schematically illustrates the covered medical device of the present invention in Example 1;
Fig. 3 shows the results of loading force tests in comparative and inventive examples;
Fig. 4 schematically illustrates the covered medical device of the present invention in Example 4;
Fig. 5 schematically illustrates the covered medical device of the present invention in Example 5;
Fig. 6 schematically illustrates the covered medical device of the present invention in Example 6; and
Fig. 7 schematically illustrates the covered medical device of the present invention in Example 7.

### List of Reference Numerals

10 main mesh stent; 101 proximal end of main mesh stent; 102 distal end of main mesh stent; 103 coupling unit; 11 trailing skirt; 20 cover; 21 distal end of cover; 210 covering membrane; 211 woven PTFE membrane; 212 woven PET membrane; 220 coating layer; 221 PTFE coating layer; 222 polyacrylamide coating layer; 223 PVDF coating layer.

### DETAILED DESCRIPTION

Objects, advantages and features of the present invention will become more apparent upon reading the following more detailed description with reference to the accompanying drawings, which illustrate specific embodiments thereof. Note that the figures are provided in a very simplified form not necessarily drawn to exact scale for the only purpose of helping to explain the disclosed embodiments in a more convenient and clearer way. In addition, the illustrated structures are usually part of their real-world counterparts. In particular, as the figures tend to have distinct emphases, they are sometimes drawn to different scales.

As used herein, the term "proximal end" usually refers to an end closer to an operator, and the term "distal end" usually refers to an end farther from the operator, during a surgical procedure. As used herein, the term "axial direction" refers to a direction running along a central axis of the covered medical device, and the "circumferential direction" refers to a direction around the central axis of the covered medical device.

It is an object of the present invention to provide a covered medical device, which can be used to treat various diseases, such as atrial septal defect (ASD), patent ductus arteriosus (PDA), ventricular septal defect (VSD) and patent foramen ovale (PFO). Preferably, the covered medical device is a left atrial appendage (LAA) occluder.

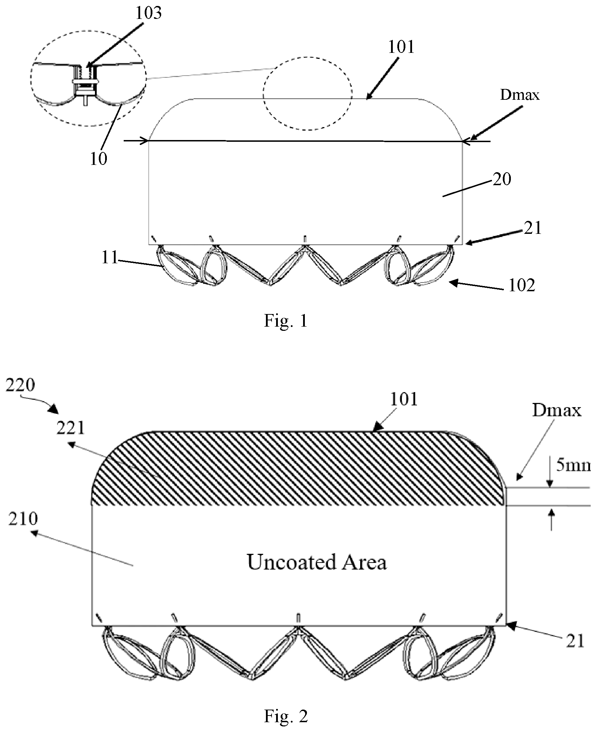

As shown in Fig. 1, the present invention provides a covered medical device including a main mesh stent 10 and a cover 20 covering at least part of an external surface of the main mesh stent 10. For example, a distal end portion of the main mesh stent 10 is not covered by the cover 20, therefore exposing a trailing skirt 11. After the main mesh stent 10 is covered on its external surface by the cover 20, an occluding disc is formed to isolate blood flow.

Typically, a height of the cover 20 does not exceed 2/3 of a total height of the main mesh stent 10. However, the present invention is not so limited. The height of the main mesh stent 10 is measured from a proximal end 101 to a distal end 102 thereof. The proximal end 101 of the main mesh stent 10 is regarded as a reference to define the height. The height of the cover 20 refers to a distance that the cover 20 extends and covers from the proximal end 101 of the main mesh stent 10 toward the distal end 102, i.e., the distance from the proximal end of the cover 20 to the distal end 21 of the cover 20. The proximal end of the cover 20 is the proximal end 101 of main mesh stent 10. A distal end of the main mesh stent 10 may extend beyond the distal end 21 of the cover 20, or the cover 20 may cover the distal end 102 of the main mesh stent 10.

The distal end 102 of the main mesh stent 10 may be open or closed, but the present invention is not so limited. In the illustrated embodiment, the distal end 102 of the main mesh stent 10 is open, and the main mesh stent 10 resembles a mesh basket. Moreover, the trailing skirt 11 disposed at the distal end 102 is folded inwardly to avoid scratching and damaging bodily tissue during and after implantation. On the main mesh stent 10, an anchoring structure may be provided somewhere between a maximum outer diameter (Dmax) position and the distal end thereof, which can pierce or hook bodily tissue to increase stability. The main mesh stent 10 is provided at the proximal end 101 with a coupling unit 103 configured for detachable attachment to a delivery system.

The main mesh stent 10 may be a cut or braided stent, with a cut stent being more preferred, because of higher stability. The main mesh stent 10 may be made of a nickel-titanium alloy, or another biocompatible material with shape-memory properties. Alternatively, it may be made of an elastically or plastically deformable material. The present invention is not limited to any particular material of the main mesh stent 10.

In addition, the cover 20 has a first friction zone and a second friction zone without any overlap therebetween arranged along an axis of the main mesh stent 10 from the proximal end 101 to the distal end 102 thereof. Both the first and second friction zones surround and cover the main mesh stent 10 so that the cover 20 surrounds and covers the circumference of the main mesh stent 10. The first friction zone has a lower coefficient of friction (static coefficient of friction) than the second friction zone.

The first friction zone extends from the proximal end 101 of main mesh stent 10 and covers the maximum outer diameter position of the main mesh stent 10. Delivery or retraction of the covered medical device may involve loading or unloading it into and from a delivery sheath. Through the first friction zone, such loading or unloading can be accomplished with reduced friction between the cover 20 and the sheath, so that the cover 20 at the maximum outer diameter (Dmax) position of the main mesh stent 10 has a lower loading force.

The second friction zone extends and covers from its boundary with the first friction zone to the distal end 21 of the cover 20. That is, the second friction zone is the rest of the cover 20 other than the first friction zone. As such, after the covered medical device is implanted into a patient's body, a friction between the cover 20 and tissue at an target object is increased by the second friction zone, and hence a post-implantation stability of the covered medical device is increased. Here, the target object refers to an implantation object, such as a blood vessel, the LAA, or a ventricular septal rupture.

According to the present invention, by the first and second friction zones of the cover 20, both good sheath loading performance and anchoring performance can be provided. A smaller force is required to load the device into a sheath, and high post-implantation anchoring strength can be attained, which results in increased safety of the device, without compromising its other performance aspects.

It will be understood that the second friction zone serves as an anchoring zone intended principally to ensure good stability of the device after it is implanted into a patient's body, allowing it to provide stable and effective occlusion at the target object. On the other hand, the first friction zone is essentially not involved in providing anchoring support to the device, and therefore has no impact on its support and anchoring strength. For example, in the case of LAA occlusion, the second friction zone may be brought into apposition with an LAA wall to ensure good support and anchoring strength, while the first friction zone may be positioned at the LAA ostium in an orientation toward the atrium, is in contact with blood and does not require anchoring.

In some embodiments, the cover 20 includes a covering membrane 210 and a coating layer 220. The covering membrane 210 directly covers the external surface of the main mesh stent 10, and the coating layer 220 directly covers the covering membrane 210. The coating layer 220 includes at least a first coefficient of friction (COF) coating layer, which forms the first friction zone. The first COF coating layer is made of a material with a lower COF than that of the covering membrane 210.

In some alternative embodiments, the cover 20 is simply made up of the covering membrane 210 and does not include the coating layer 220. In this case, the covering membrane 210 may be a combination of first and second membranes with different COFs, which are pieced together. The first membrane forms the first friction zone, and the second membrane forms the second friction zone.

In some embodiments, the coating layer 220 includes only the first COF coating layer that forms the first friction zone. In this case, the area of the covering membrane 210 other than those covered by the first COF coating layer is uncoated area, and the uncoated area directly forms the second friction zone. That is, the covering membrane 210 without coating layer forms the second friction zone.

In yet other alternative embodiments, the coating layer 220 includes the first COF coating layer that forms the first friction zone and a second COF coating layer providing the second friction zone. The area of the covering membrane 210 other than those covered by the first COF coating layer is covered by the second COF coating layer. There is no overlap between the first and second COF coating layers, and the second COF coating layer is made of a material with a higher COF than that of the first COF coating layer.

The first COF coating layer may directly cover the covering membrane 210, or directly cover at least one first transition coating layer with a higher or lower COF than the first COF coating layer. The first transition coating layer(s) can enhance to some extent the adhesion between the first COF coating layer and the covering membrane 210, reducing the risk of coating layer detachment. For example, the first transition coating layer(s) may have a higher COF than the first COF coating layer. In this case, the first transition coating layer(s) may be first applied to the corresponding area of the covering membrane 210, and the first COF coating layer may then in turn cover the first transition coating layer(s). Alternatively, the first transition coating layer(s) may have a lower COF than the first COF coating layer. In this case, similarly, the first transition coating layer(s) may be first applied to the corresponding area of the covering membrane 210, and the first COF coating layer may then in turn cover the first transition coating layer(s). In either case, it is necessary to ensure that the exposed topmost coating layer is the first COF coating layer. In practice, the first COF coating layer may be supported by one or more first transition coating layers with different COFs.

Similarly to the case of the first COF coating layer, the second COF coating layer may directly cover the covering membrane 210or directly cover at least one second transition coating layer with a higher or lower COF. For example, the second transition coating layer(s) may have a lower COF than the second COF coating layer. In this case, the second transition coating layer(s) may be first applied to the corresponding area of the covering membrane 210, and the second COF coating layer may then in turn cover the second transition coating layer(s). Alternatively, the second transition coating layer(s) may have a higher COF than the second COF coating layer. In this case, similarly, the second transition coating layer(s) may be first applied to the corresponding area of the covering membrane 210, and the second COF coating layer may then in turn cover the second transition coating layer(s). In either case, it is necessary to ensure that the exposed topmost coating layer is the second COF coating layer. Likewise, the second COF coating layer may be supported by one or more second transition coating layers with different COFs. Likewise, the second transition coating layer(s) can enhance to some extent the adhesion between the second COF coating layer and the covering membrane 210, reducing the risk of coating layer detachment.

The material of the second COF coating layer may be the same as the covering membrane 210, such as polyethylene terephthalate (PET, commonly known as Terylene), silicone or polyurethane. Alternatively, it may be made of a different polymeric material.

Preferably, the first COF coating layer includes one or a combination of a fluoropolymer, Parylene, polyimide, polyvinylpyrrolidone, polyacrylamide and a gel. More desirably, the first COF coating layer includes a fluoropolymer, which exhibits anticoagulant properties, in addition to less friction. More preferably, the first COF coating layer is made purely of a fluoropolymer.

The first COF coating layer may be made of a single material, or a combination of multiple materials with different COFs. Preferably, the first COF coating layer is made of one or more fluoropolymers, e.g., polytetrafluoroethylene (PTFE), polyvinylidene fluoride (PVDF), a vinyl fluoride homopolymer (PVF), a fluorinated or perfluorinated ethylene propylene copolymer (FEP), or a combination thereof.

Preferably, the coating layer on the covering membrane 210 has a thickness not exceeding 1 µm, in order to avoid impeding the loading of the device into a sheath.

Preferably, the first friction zone has a COF not exceeding 0.3, which imparts both less friction and anticoagulant properties. For example, in the case of a fluoropolymer, best results can be obtained if its COF does not exceed 0.1.

The COF of the second friction zone may be substantially the same as that of any suitable common material for such covering membranes. For example, the second friction zone may be formed of any suitable common material for such covering membranes.

The covering membrane 210 may be a woven or non-woven membrane, but the present invention is not so limited. In this case, the coating layer 220 may cover the entire surfaces of filaments in the covering membrane 210, or only external surfaces thereof. The coating layer 220 may be formed, for example, by dipping, spraying or otherwise.

Several exemplary examples are set forth below to further explain the present invention. These examples are presented merely for the purpose of illustration to facilitate a better understanding of the present invention by those skilled in the art, and are not intended to limit the scope of the invention in any sense.

### Example 1

As shown in Fig. 2, in Example 1, the external surface of the main mesh stent 10 is covered by a covering membrane 210, which is a woven PET membrane, and a coating layer 220 covers part of an external surface of the woven PET membrane. The coating layer 220 consists solely of a polytetrafluoroethylene (PTFE) coating layer 221 serving as a first COF coating layer, which forms the first friction zone. The PTFE coating layer 221 extends from the proximal end 101 of the main mesh stent 10 until it covers the maximum outer diameter (Dmax) position of the main mesh stent 10. For example, the PTFE coating layer 221 may extend beyond the Dmax. This ensures that the PTFE coating layer 221 can fully cover the maximum outer diameter (Dmax) position of the main mesh stent 10. The PTFE coating layer 221 may extend a distance of 5 mm or the like beyond the Dmax. The rest of the covering membrane 210 that is not covered by the PTFE coating layer 221 is an uncoated area that directly forms the second friction zone.

COFs of the PTFE coating layer 221 and the uncoated portion were measured using a COF tester according to a method specified in GB/T 10006-2021. The results are summarized in Table 1 below.

**Table 1 COFs of PTFE Coating layer and Uncoated Area**

| Area | Static COF |
|---|---|
| PTFE Coating layer | 0.1 |
| Uncoated Area (Woven PET Membrane) | 0.5 |

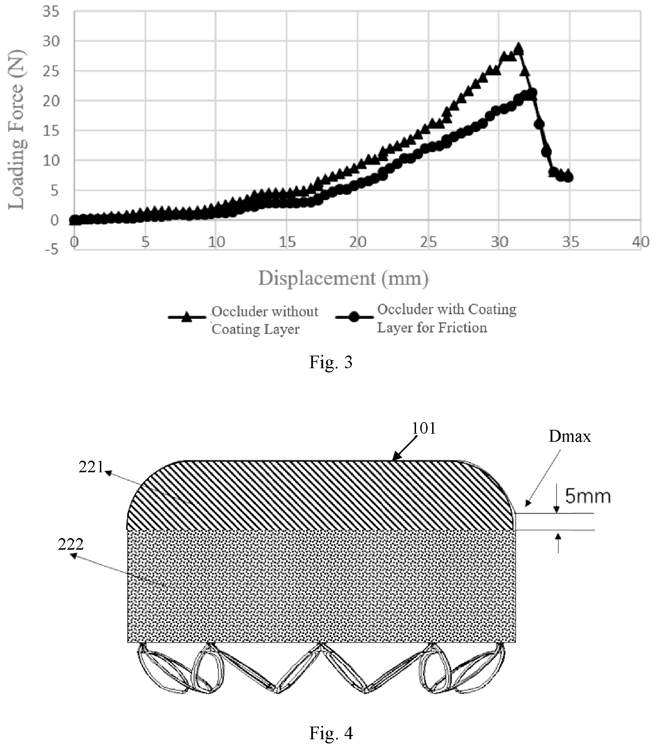

The loading force was further tested. During the test, the covered medical device of Example 1 and the covered medical device of the comparative example were loaded into a 12F sheath, and the loading force (in N) required for each covered medical device as a function of sheath displacement was measured. The measurement results are shown in Figure 3. It should be noted that in a comparative example, the woven PET membrane is not provided thereon with any coating layer, i.e., the entire external surface of the woven PET membrane was an uncoated area. It should also be understood that the maximum loading force corresponds to the force applied when the device's maximum outer diameter is pulled into the sheath, and the force at the maximum outer diameter is recorded as the loading force.

As can be seen from Fig. 3, the loading forces in Example 1 and the comparative example are approximately 20 N and approximately 28.6 N, respectively. Therefore, the presence of the PTFE coating layer 221 on the woven PET membrane leads to a reduced loading force.

### Example 2

Example 2 differs from Example 1 in that the first friction zone is formed by a polyvinylidene fluoride (PVDF) coating layer, instead of the PTFE coating layer 221 in Example 1. Except for this difference, Example 2 is the same as Example 1, and further detailed description is omitted.

COFs of the PVDF coating layer and the uncoated area were measured in the same way. The results are summarized in Table 2 below.

**Table 2 COFs of PVDF Coating layer and Uncoated Area**

| Area | Static COF |
|---|---|
| PVDF Coating layer | 0.07 |
| Uncoated Area (Woven PET Membrane) | 0.5 |

As can be seen from Table 2, the COF of the PVDF coating layer (0.07) is lower than that of the PTFE coating layer 221 (0.1). Accordingly, a loading force is even smaller, and the detailed loading force measurements are shown in Table 3.

**Table 3 Maximum Loading Forces under Different Test Conditions**

| Object Under Test | Maximum Loading Force |
|---|---|
| Example 2 (PVDF Coating layer + Uncoated Area) | 18.5 N |
| Comparative Example (Woven PET Membrane without Coating layer) | 28.6 N |

As can be seen from Table 3, the maximum loading forces in Example 2 and the comparative example are 18.5 N and 28.6 N, respectively. Therefore, applying PVDF coating layer on the woven PET membrane, the maximum loading force is even smaller. Moreover, the PVDF coating layer possesses anticoagulant properties, which can lower the likelihood of platelet deposition onto the device and hence of thrombosis induced by the device.

### Example 3

Example 3 differs from Example 1 in that the first friction zone is formed by a Parylene coating layer, instead of the PTFE coating layer 221 in Example 1, and that the Parylene coating layer extends about 3 mm beyond the maximum outer diameter of the main mesh stent 10. Except for these differences, Example 3 is the same as Example 1, and further detailed description is omitted.

The results of tests using a COF tester are summarized in Table 4 below.

**Table 4 COFs of Parylene Coating layer and Uncoated Area**

| Area | Static COF |
|---|---|
| Parylene Coating layer | 0.05 |
| Uncoated Area(Woven PET Membrane) | 0.5 |

As shown in Table 4, the Parylene coating layer has a lower COF than both the PVDF coating layer and the PTFE coating layer 221. Accordingly, the maximum loading force is even smaller, and the loading force measurements are shown in Table 5.

**Table 5 Maximum Loading Forces under Different Test Conditions**

| Object Under Test | Maximum Loading Force |
|---|---|
| Example 3 (Parylene Coating layer + Uncoated Area) | 15.0 N |
| Comparative Example (Woven PET Membrane without Coating layer) | 28.6 N |

As can be seen, the maximum loading force is reduced to 15.0 N in Example 3, resulting in lower loading difficulty and reduced loading resistance.

### Example 4

As shown in Fig. 4, in Example 4, the coating layer 220 further includes a polyacrylamide coating layer 222 serving as a second COF coating layer, which forms the second friction zone. The polyacrylamide coating layer 222 covers the rest of the woven PET membrane other than the area covered by the PTFE coating layer 221.

COFs of the PTFE coating layer 221 and the polyacrylamide coating layer 222 were measured in the same way. The results are summarized in Table 6 below.

**Table 6 COFs of PTFE and Polyacrylamide Coating layers**

| Area | Static COF |
|---|---|
| PTFE Coating layer | 0.1 |
| Polyacrylamide Coating layer | 0.15 |

As can be seen, the COF of the polyacrylamide coating layer 222 (0.15) is higher than that of the PTFE coating layer 221 (0.1), allowing the coating layer 220 to achieve both high and low friction zones. Loading force measurements are presented in Table 7.

**Table 7 Loading Forces under Different Test Conditions**

| Object Under Test | Maximum Loading Force |
|---|---|
| Example 4 (PTFE Coating layer + Polyacrylamide Coating layer) | 20.0 N |
| Comparative Example (Woven PET Membrane without Coating layer) | 28.6 N |

As can be seen, in comparison to the device without a coating layer, a reduced loading force can also be achieved by providing the first and second COF coating layers on the woven PET membrane.

### Example 5

Example 5 differs from Examples 1 to 4 in that the cover 20 consists solely of a covering membrane 210 and does not include a coating layer. Instead, the covering membrane 210 is a combination of first and second membranes with different COFs, which are pieced together. The first membrane forms the first friction zone, and the second membrane forms the second friction zone.

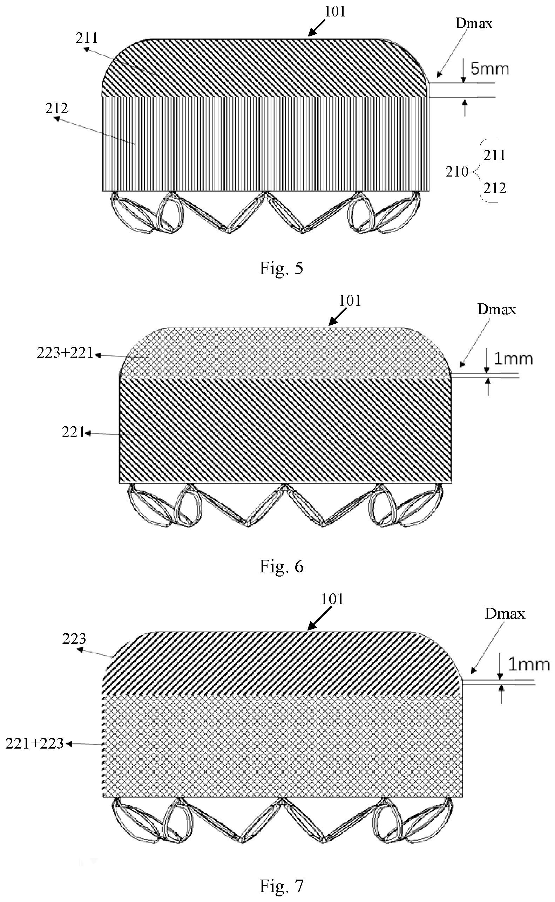

Specifically, as shown in Fig. 5, the covering membrane 210 is composed of a PTFE membrane 211 and a woven PET membrane 212, which are pieced together. The PTFE membrane 211 serves as the first membrane, and the woven PET membrane 212 as the second membrane. The PTFE membrane 211 extends from the proximal end 101 of main mesh stent 10 until it covers the maximum outer diameter position of the main mesh stent 10. More desirably, the PTFE membrane 211 extends about 5 mm beyond the maximum outer diameter position. The rest of the main mesh stent 10 other than the area covered by the PTFE membrane 211 is covered by the woven PET membrane 212.

COFs of the woven PTFE membrane 211 and the woven PET membrane 212 are measured using a COF tester. The results are summarized in Table 8 below.

**Table 8 COFs of Woven PTFE and PET Membranes**

| Area | Static COF |
|---|---|
| PTFE Membrane (Low Friction Zone) | 0.15 |
| Woven PET Membrane (High Friction | 0.5 |
| Zone) | |

The maximum loading force was further tested. During the test, the covered medical device of Example 5 and the covered medical device of the comparative example were loaded into a 12F sheath, and the maximum loading forces were measured. The results are summarized in Table 9 below.

**Table 9 Loading Forces under Different Test Conditions**

| Object Under Test | Maximum Loading Force |
|---|---|
| Example 5 (PTFE Membrane + Woven PET Membrane) | 20.0 N |
| Comparative Example (Woven PET Membrane without Coating layer) | 28.6 N |

As shown in Table 9, the covering membrane 210 with different COFs also reduces a loading force of the entire device, as expected.

### Example 6

Example 6 differs from the preceding example in that the first COF coating layer covers a first transition coating layer with a higher COF than the first COF coating layer. Specifically, as shown in Fig. 6, the external surface of the main mesh stent 10 is covered with a woven PET membrane (serving as a covering membrane 210). A PTFE coating layer 221 is applied to the entire surface of the woven PET membrane, and a PVDF coating layer 223 is then applied to an area of the PTFE coating layer 221 where the first friction zone is required to be formed. In this way, the first friction zone is provided by a composite coating layer. Since the PVDF coating layer 223 has a lower COF than the PTFE coating layer 221, a relatively low COF of the surface of the first friction zone of the cover can be still assured.

In this example, the PVDF coating layer 223 extends about 1 mm beyond the maximum outer diameter of the main mesh stent 10. However, it will be understood that the present invention is not limited to any particular distance the first COF coating layer extends beyond the maximum outer diameter of the main mesh stent 10. Indeed, any distance is possible, as long as the maximum outer diameter position of the main mesh stent 10 can be fully covered by the lower COF coating layer.

The rest of the PTFE coating layer 221 other than the area covered by the PVDF coating layer 223 is not further treated, and the exposed PTFE coating layer 221directly forms the second friction zone.

COF and loading force measurements in Example 6 are summarized in Tables 10 and 11, respectively.

**Table 10 COFs of PTFE Coating layer and PVDF Coating layer on PTFE Coating layer**

| Area | Static COF |
|---|---|
| PVDF Coating layer covers PTFE Coating layer | 0.07 |
| PTFE Coating layer Alone | 0.1 |

**Table 11 Maximum Loading Forces under Different Test Conditions**

| Object Under Test | Maximum Loading Force |
|---|---|
| Example 6 (PVDF Coating layer covers PTFE Coating layer) | 18.3 N |
| Comparative Example (Woven PET Membrane without Coating layer) | 28.6 N |

As can be seen from Tables 10 and 11, a composite coating layer is formed in low friction zone and the high friction zone is a single coating layer, the loading force also can be reduced, and same or similar effect is achieved.

### Example 7

Example 7 differs from Example 6 in that the second COF coating layer covers a second transition coating layer with a lower COF than the second COF coating layer.

As shown in Fig. 7, a PVDF coating layer 223covers the entire surface of the woven PET membrane, and a PTFE coating layer 221 then cover an area of the PVDF coating layer 223 where the second friction zone is required to be formed. In this way, the second friction zone is provided by a composite coating layer. Likewise, since the PTFE coating layer 221 has a higher COF than the PVDF coating layer 223, a relatively high COF of the surface of the second friction zone of the cover can be still assured.

The rest of the PVDF coating layer 223 other than the area covered by the PTFE coating layer 221 is not further treated, and the exposed PVDF coating layer 223 directly forms the first friction zone.

COF and maximum loading force measurements in Example 7 are summarized in Tables 12 and 13, respectively.

**Table 12 COFs of PVDF Coating layer and PTFE Coating layer on PVDF Coating layer**

| Area | Static COF |
|---|---|
| PVDF Coating layer Alone (Low Friction Zone) | 0.07 |
| PTFE Coating layer covers PVDF Coating layer (High Friction Zone) | 0.1 |

**Table 13 Maximum Loading Forces under Different Test Conditions**

| Object Under Test | Maximum Loading Force |
|---|---|
| Example 7 | 18.5 N |
| Comparative Example (Woven PET Membrane without Coating layer Alone) | 28.6 N |

As can be seen from Tables 12 and 13, a composite coating layer is formed in high friction zone and the low friction zone is a single coating layer, the loading force also can be reduced, and same or similar effect is achieved.

### Comparative Example 1

Differing from Example 1, a woven PET membrane is entirely covered by a PTFE coating layer 221 so that the whole surface of the cover 20 is the first friction zone.

The pull-out force was further measured, and the pull-out forces of the devices of Example 1 and Comparative Example 1 were tested in a pull-out force test model made of silicone. The results are summarized in Table 14 below.

**Table 14 Pull-off Forces under Different Test Conditions**

| Device | Pull-off Force |
|---|---|
| Example 1 | 2.5 N |
| Comparative Example 1 | 0.8 N |

As can be seen from Table 14, although the loading force of the device of Comparative Example 1 is expected to be consistent with that of Example 1, its pull-off force is too small. Therefore, after implantation into the human body, the device has a risk of detachment, and its stability cannot be guaranteed. In contrast, the device of Example 1 achieves both good loading force and anchoring performance, yielding better results.

In summary, the covered medical device provided by the present invention can match the functions of different areas of the device through a high-and-low friction zone design, thereby achieving better clinical outcomes. The first friction zone, which covers the maximum outer diameter position, requires a smaller loading force during sheathing, offering greater design freedom for the device and facilitating the design of smaller-diameter sheaths or larger-sized devices. The area extending from the maximum outer diameter position toward the distal end serves as the anchoring area, where a high COF provides better device stability. Particularly when the first friction zone employs a fluoropolymer coating layer, an even lower surface COF can be achieved, resulting in an even smaller loading force, while also reducing the probability of platelet deposition and lowering the incidence of device-induced thrombosis, thus leading to better overall performance.

Further, it should be noted that, in the device of the present invention, although the maximum outer diameter position of the main mesh stent is covered by the low friction zone (i.e., the first friction zone), as the device essentially relies on the high friction zone (i.e., the second friction zone) for stable anchoring, the low friction zone does not adversely affect the overall stability of the device.

The description presented above is merely that of some preferred embodiments of the present invention and does not limit the scope thereof in any sense. Any and all changes and modifications made by those of ordinary skill in the art based on the above teachings fall within the scope as defined in the appended claims.

## Claims

1. A covered medical device, comprising a main mesh stent and a cover, wherein the cover covers at least a portion of an external surface of the main mesh stent, wherein the cover comprises a first friction zone and a second friction zone that are arranged along an axis of the main mesh stent from a proximal end to a distal end, wherein the first friction zone has a coefficient of friction (COF) lower than a COF of the second friction zone, and wherein the first friction zone extends from the proximal end of the main mesh stent and covers a maximum outer diameter position of the main mesh stent.

2. The covered medical device according to claim 1, wherein the cover comprises a covering membrane and a coating layer, wherein the covering membrane covers the external surface of the main mesh stent, wherein the coating layer covers the covering membrane, and wherein the coating layer comprises a first COF coating layer that forms the first friction zone.

3. The covered medical device according to claim 2, wherein an area of the covering membrane that is not covered by the first COF coating layer is an uncoated area, and wherein the uncoated area forms the second friction zone.

4. The covered medical device according to claim 2, wherein the coating layer further comprises a second COF coating layer that forms the second friction zone, wherein an area of the covering membrane that is not covered by the first COF coating layer is covered by the second COF coating layer.

5. The covered medical device according to claim 2, wherein the first COF coating layer covers the covering membrane, or wherein the first COF coating layer covers at least one first transition coating layer with a higher or a lower COF than the first COF coating layer.

6. The covered medical device according to claim 4, wherein the second COF coating layer covers the covering membrane, or wherein the second COF coating layer covers at least one second transition coating layer with a higher or lower COF than the second COF coating layer.

7. The covered medical device according to claim 2, wherein the first COF coating layer comprises one or a combination of a fluoropolymer, Parylene, polyimide, polyvinylpyrrolidone, polyacrylamide, poly(ether amide) and a gel.

8. The covered medical device according to claim 7, wherein the fluoropolymer comprises one or a combination of polytetrafluoroethylene, polyvinylidene fluoride, a vinyl fluoride homopolymer and a perfluorinated ethylene propylene copolymer.

9. The covered medical device according to claim 2, wherein the coating layer has a thickness not exceeding 1 µm.

10. The covered medical device according to claim 1, wherein the COF of the first friction zone does not exceed 0.3.

11. The covered medical device according to claim 10, wherein the COF of the first friction zone does not exceed 0.1.

12. The covered medical device according to claim 1, wherein the cover consists of a covering membrane, wherein the covering membrane is composed of a first membrane and a second membrane that have different COFs and are pieced together, wherein the first membrane forms the first friction zone, and wherein the second membrane forms the second friction zone.
